# EUROPÄISCHE PATENTSCHRIFT

(11) **EP 1 879 846 B1**
(45) Veröffentlichungstag und Bekanntmachung des Hinweises auf die Patenterteilung: **15.07.2009**
(21) Anmeldenummer: 06763057.4
(22) Anmeldetag: 26.04.2006
(51) Int. Cl.: C07C 51/44, C07C 51/50, C07C 57/04

(54) **VERFAHREN DER REKTIFIKATIVEN AUFTRENNUNG EINER ACRYLSÄURE UND/ODER METHACRYLSÄURE ENTHALTENDEN FLÜSSIGKEIT**
PROCESS FOR SEPARATING BY RECTIFICATION A LIQUID WHICH CONTAINS AN ACRYLIC ACID AND/OR A METHACRYLIC ACID
PROCEDE DE SEPARATION PAR RECTIFICATION D'UN LIQUIDE CONTENANT DE L'ACIDE ACRYLIQUE ET/OU DE L'ACIDE METHACRYLIQUE

(30) Priorität: 27.04.2005 DE 102005019911; 27.04.2005 US 675087 P
(43) Veröffentlichungstag der Anmeldung: 23.01.2008
(73) Patentinhaber: BASF SE, 67056 Ludwigshafen (DE)
(72) Erfinder: LIPOWSKY, Gunter, 69198 Schriesheim (DE); MÜLLER-ENGEL, Klaus, Joachim, 76297 Stutensee (DE)
(86) Internationale Anmeldenummer: PCT/EP2006/061843
(87) Internationale Veröffentlichungsnummer: WO 2006/114428

(56) Entgegenhaltungen:
- EP-A- 1 041 062
- WO-A-20/05035478
- US-A- 4 113 574
- US-A1- 2002 002 253
- US-A1- 2003 208 093
- US-A1- 2004 225 151

## Beschreibung

Vorliegende Erfindung betrifft ein Verfahren der rektifikativen Auftrennung einer Flüssigkeit II, deren Gehalt an Acrylsäure und/oder Methacrylsäure, bezogen auf das Gesamtgewicht der Flüssigkeit II, wenigstens 10 Gew.-% beträgt, und die neben Acrylsäure und/oder Methacrylsäure sowohl Acrolein und/oder Methacrolein als auch Aceton in einer Gesamtmenge von nicht mehr als 5 Gew.-%, bezogen auf die in der Flüssigkeit II enthaltene Menge an Acrylsäure und/oder Methacrylsäure mit der Maßgabe enthält, dass das Gewichtsverhältnis von in der Flüssigkeit II enthaltenem Acrolein zu in der Flüssigkeit II enthaltenem Aceton von 3,5 verschieden ist, und die erzeugt wurde, ohne einer anderen Acrylsäure und/oder Methacrylsäure enthaltenden Flüssigkeit I Acrolein oder Methacrolein als Reinsubstanz zuzusetzen.

Acrylsäure und Methacrylsäure, entweder als solche oder in Form ihrer Ester, sind insbesondere zur Herstellung von Polymerisaten für die verschiedensten Anwendungen, z.B. Verwendung als Klebstoffe, Verwendung als Wasser superabsorbierende Materialien, von Bedeutung und weisen insbesondere in flüssiger Phase eine hohe Neigung zu radikalischer Polymerisation auf. Sichere Lagerung von Acrylsäure und/oder Methacrylsäure enthaltenden Flüssigkeiten ist selbst bei tiefen Temperaturen nur unter Zusatz von Polymerisationsinhibitor möglich. Unter anderem sind Acrylsäure und Methacrylsäure durch partielle heterogen katalysierte Gasphasenoxidation von Vorläuferverbindungen wie Alkanen, Alkanolen, Alkenen und/oder Alkenalen erhältlich, die 3 bzw. 4 C-Atome enthalten (oder aus einem Gemisch aus diesen). Acrylsäure ist vorteilhaft durch partielle heterogen katalysierte Gasphasenoxidation von Propan, Propen und/oder Acrolein erhältlich. Methacrylsäure ist vorteilhaft durch partielle heterogen katalysierte Gasphasenoxidation von tert.-Butanol, iso-Buten, iso-Butan, iso-Butyraldehyd und/oder Methacrolein erhältlich. Als Ausgangsverbindungen sind aber auch solche denkbar, aus welchen sich die eigentliche C₃-/C₄-Ausgangsverbindung während der partiellen heterogen katalysierten Gasphasenoxidation erst intermediär bildet. Beispielhaft genannt sei der Methylether des tert.-Butanols.

Dabei werden die genannten Ausgangsgase in der Regel mit inerten Gasen wie Stickstoff, CO₂, anderen gesättigten Kohlenwasserstoffen, CO, Edelgas (He, Ar etc.) und/oder Wasserdampf verdünnt, im Gemisch mit Sauerstoff bei erhöhten Temperaturen (üblicherweise 200 bis 400 bzw. bis 450°C) sowie gegebenenfalls auf über Normaldruck erhöhtem Druck über vorzugsweise übergangsmetallische (z.B. Mo, Fe und Bi bzw. Mo und V bzw. Mo und P enthaltende) Mischoxidkatalysatoren geleitet und oxidativ in Acrylsäure und/oder Methacrylsäure umgewandelt (vgl. z.B. DE-A 44 05 059, EP-A 253 409, EP-A 092 097, DE-A 44 31 949, EP-A 990 636, EP-A 1 106 598, EP-A 1 192 987, EP-A 529 853, EP-A 1 350 784, DE-A 198 55 913 und DE-A 101 01 695 sowie der in diesen Schriften zitierte Stand der Technik).

In der Regel erfolgt die Herstellung von Acrylsäure aus C₃-Vorläufern getrennt von der Herstellung von Methacrylsäure, bei deren Herstellung man von C₄-Vorläufern ausgeht. Geht man von einem Gemisch aus C₃- und C₄-Vorläufern aus, können Acryl- und Methacrylsäure aber auch im Gemisch durch partielle gasphasenkatalytische Oxidation hergestellt werden.

Aufgrund zahlreicher im Verlauf der heterogen katalysierten partiellen Gasphasenoxidation erfolgender Parallel- und Folgereaktionen sowie aufgrund der mitzuverwendenden inerten Verdünnungsgase und der in den Roh-Alkanen, -Alkanolen, -Alkenen und/oder-Alkenalen in der Regel enthaltenen Verunreinigungen (vgl. z.B. DE-A 102 45 585, WO 01/96270 und WO 03/011804 (z.B. Verwendung von polymer grade oder von chemical grade Propylen)) wird im Rahmen der heterogen katalysierten partiellen Gasphasenoxidation jedoch nicht nur Acrylsäure und/oder Methacrylsäure, sondern ein Reaktionsgasgemisch erhalten, das neben Acrylsäure und/oder Methacrylsäure und den inerten Verdünnungsgasen Nebenprodukte enthält, von denen die als Zielprodukt angestrebte Acrylsäure und/oder Methacrylsäure abgetrennt werden muss.

Üblicherweise erfolgt die Abtrennung der Acrylsäure und/oder Methacrylsäure aus dem Reaktionsgasgemisch der heterogen katalysierten partiellen Gasphasenoxidation dadurch, dass man zunächst in einer Grundabtrennung die Acrylsäure und/oder Methacrylsäure aus der Gasphase in die flüssige Phase überführt.

Dies kann z.B. dadurch erfolgen, dass das Produktgasgemisch der heterogen katalysierten partiellen Gasphasenoxidation einem Kondensationsschritt unterworfen wird, der durch direkte und/oder indirekte Kühlung ausgelöst werden kann. Vorzugsweise wird der Kondensationsschritt fraktionierend ausgeführt (vgl. z.B. DE-A 199 24 532, DE-A 199 24 533, DE-A 197 40 253, DE-A 196 27 847 und DE-A 103 32 758).

Alternativ kann die Überführung der Acrylsäure und/oder Methacrylsäure in die kondensierte Phase auch dadurch erfolgen, dass man sie aus dem Reaktionsgasgemisch der heterogen katalysierten partiellen Gasphasenoxidation heraus in ein geeignetes Absorptionsmittel absorbiert (vgl. z.B. US 2004/0242826, DE-A 196 06 877, DE-A 196 31 645, EP-A 982 289, EP-A 982 288, EP-A 982 287, EP-A 792 867, EP-A 784 046, DE-A 103 36 386, DE-A 43 08 087, DE-A 21 36 396, EP-A 648 732 (inbesondere das Ausführungsbeispiel), EP-A 1 125 912, EP-A 1 212 280).

Als Absorptionsmittel kommen dabei z.B. Wasser, wässrige Lösungen und organische Lösungsmittel in Betracht. Als organische Absorptionsmittel werden dabei solche bevorzugt, deren Siedepunkt bei Normalbedingungen (1 bar) oberhalb des Siedepunktes von Acrylsäure und/oder Methacrylsäure liegt und die vorzugsweise vergleichsweise hydrophob sind, um eine Mitabsorption von Reaktionswasser weitestgehend zu vermeiden.

Solchermaßen geeignete organische Absorptionsmittel sind z.B. Mischung aus 70 bis 75 Gew.-% Diphenylether und 25 bis 30 Gew.-% Diphenyl sowie Mischungen aus 0,1 bis 25 Gew.-% o-Dimethylphthalat und 75 bis 99,9 Gew.-% eines vorgenannten Diphenylether/Diphenyl Gemischs (vgl. z.B. DE-A 43 08 087 und DE-A 21 36 396).

Selbstredend können Absorption und Kondensation auch kombiniert angewendet werden, wie es z.B. die EP-A 784 046 sowie die DE-A 44 36 243 beschreiben.

Aus dem Kondensat oder Absorbat können leichtsiedende Nebenkomponenten je nach Zielsetzung mittels Desorption und/oder Strippen mittels Gasen wie Stickstoff oder Luft entfernt werden. Nachfolgend kann die Acrylsäure und/oder Methacrylsäure über rektifikative Trennsequenzen aus der verbliebenen kondensierten Phase in beliebiger Reinheit abgetrennt werden. Selbstverständlich kann die rektifikative Abtrennung der Acrylsäure und/oder Methacrylsäure auch unmittelbar aus dem Absorbat bzw. Kondensat heraus vorgenommen werden. Wird die Überführung aus der Gasphase in die kondensierte Phase so durchgeführt, dass diese die Acrylsäure und/oder Methacrylsäure vergleichsweise verdünnt enthält (z.B. nur zu 30 Gew.-%, oder nur zu 20 Gew.-%, oder nur zu 10 Gew.-%), wird im Stand der Technik auch empfohlen, die Acrylsäure und/oder Methacrylsäure aus der unmittelbar anfallenden kondensierten Phase heraus durch Extraktion mittels eines geeigneten Extraktionsmittels (vor oder nach einer Desorption und/oder Strippung) in selbiges anzureichern (vgl. z.B. Patentschrift 54 354 der Deutschen Demokratischen Republik und EP-A 312 191) und nachfolgend die rektifikative Abtrennung der Acrylsäure und/oder Methacrylsäure ausgehend vom Extrakt vorzunehmen.

Wurde eine wässrige Phase als Absorptionsmittel verwendet, kann ein erster rektifikativer Trennschritt auch darin bestehen, mit Hilfe eines azeotropen Schleppmittels Wasser aus der kondensierten Phase heraus abzutrennen (vgl. z.B. US 2004/0242286, EP-A 695 736, EP-A 778 255, EP-A 1 041 062, EP-A 1 070 700).

Nachteilig an den beschriebenen Verfahrensweisen ist, dass das Reaktionsgasgemisch der heterogen katalysierten Gasphasenpartialoxidation im wesentlichen unabdingbar noch Reste des Vorläuferaldehyds der Acrylsäure und/oder Methacrylsäure, d.h. Acrolein und/oder Methacrolein enthält. Dies ist darauf zurückzuführen, dass die heterogen katalysierte gasphasenkatalytisch oxidative Herstellung von Acrylsäure und/oder Methacrylsäure grundsätzlich über den entsprechenden Vorläuferaldehyd als Vorstufe verläuft (vgl. z.B. JP-A 7-10802).

Dies ist insofern von Nachteil, als z.B. gemäß der DE-A 195 39 295 die Polymerisationsneigung sowohl von Acrylsäure als auch von Methacrylsäure im Beisein selbst geringster Mengen ihres Vorläuferaldehyds (im ppm-Bereich) signifikant erhöht ist. Dies ist vor allem deshalb nachteilig, weil normalerweise auch die vorstehend beschriebenen, Acrylsäure und/oder Methacrylsäure enthaltenden, kondensierten Phasen im Regelfall geringe Mengen des entsprechenden Vorläuferaldehyds enthalten (je nach Verunreinigung des für die Partialoxidation eingesetzten Rohstoffs können sowohl im Fall der Acrylsäure als auch im Fall der Methacrylsäure beide Vorläuferaldehyde, d.h., Acrolein und Methacrolein, enthalten sein), was vor allem unter der thermischen Belastung einer Rektifikation unter anderem ursächlich dafür ist, dass die rektifikative Auftrennung von Flüssigkeiten, die neben Methacrylsäure und/oder Acrylsäure auch Acrolein und/oder Methacrolein enthalten, von Zeit zu Zeit wegen unerwünschter Polymerisatbildung unterbrochen werden muss.

Als Gegenmaßnahme wird im Stand der Technik empfohlen, sowohl die Bedingungen der heterogen katalysierten Gasphasenpartialoxidation als auch die Überführung der Acrylsäure und/oder Methacrylsäure aus der Gasphase in die flüssige Phase und die Behandlung der letzteren vorab einer sich anschließenden rektifikativen Auftrennung so durchzuführen, dass die rektifikativ zu behandelnde Acrylsäure und/oder Methacrylsäure enthaltende, Flüssigkeit möglichst wenig Acrolein und/oder Methacrolein sowie möglichst wenig sonstige aldehydische Nebenprodukte, wie z.B. Formaldehyd, Glyoxal, Acetaldehyd, Propionaldehyd, n-Butyraldehyd, iso-Butyraldehyd, Furfural, Benzaldehyd und Crotonaldehyd (die alle gleichfalls polymerisationsfördernd wirken; allerdings kommt den Vorläuferaldehyden (auch aufgrund ihrer physikalischen Beschaffenheit) hinsichtlich Menge und Wirkung die größte Bedeutung zu) enthält. Diesbezüglich in geeigneter Weise zu wählende Einflussgrößen sind die Auswahl der Multimetalloxidkatalysatoren, die Wahl des Rohstoffs, die Wahl der Reaktionstemperatur, die Wahl des Umsatzes, die Wahl des Verdünnungsgrades im Reaktionsgasgemisch, die Wahl des Absorptionsmittels, die Wahl der Absorptionsbedingungen.

Alle vorgenannten Maßnahmen sind jedoch mit Nachteilen behaftet. So sind insbesondere reine Rohstoffe vergleichsweise teuer.

Hohe Umsätze der aldehydischen Vorläuferverbindung zum Zielprodukt Acrylsäure und/oder Methacrylsäure gehen üblicherweise in notwendiger Weise mit einem erhöhten Anteil an Vollverbrennung zu CO₂ und H₂O einher. Desorptive und/oder mittels Leichtsiederstrippung bewirkte Minderung des Aldehydgehaltes bedingt normalerweise Verluste an Acrylsäure und/oder Methacrylsäure. Hochselektive Multimetalloxidkatalysatoren sind in der Regel vergleichsweise aufwendig in ihrer Herstellung.

Alternativ wird im Stand der Technik empfohlen, die aldehydischen Nebenkomponenten durch eine rektifikative Vorabbehandlung mittels Verbindungen, die eine Amingruppe aufweisen, chemisch zu binden (vgl. z.B. EP-A 312 191, DE-A 22 07 184, DE-A 195 39 295, EP-A 270 999, DE-A 196 34 614).

Nachteilig an dieser Verfahrensweise ist jedoch, dass sie eines zusätzlichen Wirkstoffs bedarf.

WO 2005/035478 beschreibt die rektifikative Auftrennung einer Acrylsäure enthaltenden Flüssigkeit, wobei die Anwesenheit von mindestens 500 Gew%-ppm an Diacrylsäure polymerisationsinhibierend wirkt. In US-A 4 113 574 wird die polymerisationsinhibierende Wirkung von Acetylaceton auf (Meth)acrylsäure und -ester beschrieben.

In der EP-A 1 041 062 wird erstmals die Rolle einer weiteren Nebenkomponente angesprochen, die sowohl bei der gasphasenkatalytisch oxidativen Herstellung von Acrylsäure aus C₃-Vorläuferverbindungen (insbesondere Propylen, Acrolein und/oder Propan) als auch bei der gasphasenkatalytisch oxidativen Herstellung von Methacrylsäure aus C₄-Vorläuferverbindungen (insbesondere iso-Buten, Methacrolein, tert.-Butanol, iso-Butyraldehyd, iso-Butan und/oder der Methylether des tert.-Butanols) unabdingbar mitgebildet wird. Diese Nebenkomponente ist Aceton.

Gemäß der Lehre der EP-A 1 041 062 sowie der zugehörigen Einspruchsakte wird das Aceton zu den Vorläuferaldehyden Acrolein und/oder Methacrolein wirkungsgleich postuliert. D.h., es wird unterstellt, dass ein Beisein von Aceton in gleicher Weise wie ein Beisein von Acrolein und/oder Methacrolein in kondensierte Phase in unerwünschter Weise die Neigung von Acrylsäure und/oder Methacrylsäure zu radikalischer Polymersiations fördert. Dabei wird diesbezüglich sogar ein synergistisches Zusammenwirken von Aceton und den Vorläuferaldehyden angenommen.

Entsprechend aussagekräftige experimentelle Untersuchungen enthält die EP-A 1 041 062 allerdings nicht. Es wird lediglich empfohlen, die Gesamtmenge aus Aceton und C₂- bis C₄-Aldehyden (insbesondere Acrolein und Methacrolein) vorab einer rektifikativen Auftrennung weitestgehend abzusenken und die Wirkung einer solchen gemeinsamen Absenkung verifiziert.

In ausgedehnten eigenen Untersuchungen wurde nun überraschend gefunden, dass sich Aceton, insbesondere im Beisein der Vorläuferaldehyde, in kondensierter Phase nicht nur nicht fördernd auf die Neigung von Acrylsäure und/oder Methacrylsäure zu radikalischer Polymerisation auswirkt. Vielmehr kann Aceton im vorstehenden Kontext sogar eine polymerisationsinhibierende Wirkung zugeschrieben werden.

Demgemäß wird erfindungsgemäß ein verbessertes Verfahren der rektifikativen Auftrennung einer Flüssigkeit II, deren Gehalt an Acrylsäure und/oder Methacrylsäure, bezogen auf das Gesamtgewicht der Flüssigkeit II, wenigstens 10 Gew.-% beträgt, und die neben Methacrylsäure und/oder Acrylsäure sowohl Acrolein und/oder Methacrolein als auch Aceton in einer Gesamtmenge von nicht mehr als 5 Gew.-%, bezogen auf die in der Flüssigkeit II enthaltene Menge an Acrylsäure und/oder Methacrylsäure mit der Maßgabe enthält, dass das Gewichtsverhältnis von in der Flüssigkeit II enthaltenem Acrolein zu in der Flüssigkeit 11 enthaltenem Aceton von 3,5 verschieden ist und die erzeugt wurde, ohne einer anderen Acrylsäure und/oder Methacrylsäure enthaltenden Flüssigkeit I Acrolein oder Methacrolein als Reinsubstanz zuzusetzen, gefunden, das dadurch gekennzeichnet ist, dass man die Flüssigkeit II mit der Maßgabe in eine Rektifikationskolonne führt, dass sie, bezogen auf in der Flüssigkeit II enthaltenes Acrolein und Methacrolein, wenigstens 10 Gew.-% Aceton enthält.

Erfindungsgemäß bevorzugt enthält die der Rektifikationskolonne zugeführte Flüssigkeit II, bezogen auf die in ihr enthaltene Gesamtmenge an Acrolein und Methacrolein, wenigstens 15 Gew.-%, mit Vorteil wenigstens 20 Gew.-%, vorteilhafter wenigstens 25 Gew.-%, besser wenigstens 30 Gew.-%, noch besser wenigstens 35 Gew.-%, mit Vorzug wenigstens 40 Gew.-%, besonders bevorzugt wenigstens 45 Gew.-% und ganz besonders bevorzugt wenigstens 50 Gew.-% an Aceton.

D.h., die der Rektifikationskolonne zugeführte Flüssigkeit II, kann beim erfindungsgemäßen Verfahren, bezogen auf die in ihr enthaltene Gesamtmenge an Acrolein und Methacrolein, wenigstens 55 Gew.-%, oder wenigstens 60 Gew.-%, oder wenigstens 65 Gew.-%, oder wenigstens 70 Gew.-%, oder wenigstens 75 Gew.-%, oder wenigstens 80 Gew.-%, oder wenigstens 85 Gew.-%, oder wenigstens 90 Gew.-%, oder wenigstens 95 Gew.%, oder wenigstens 100 Gew.-% oder mehr (z.B. wenigstens 110 Gew.-%, oder wenigstens 120 Gew.-%, oder wenigstens 130 Gew.-%, oder wenigstens 140 Gew.-%, oder wenigstens 150 Gew.%, oder wenigstens 170 Gew.-%, oder wenigstens 200 Gew.-%, oder wenigstens 300 Gew.-%) an Aceton enthalten.

In der Regel wird die beim erfindungsgemäßen Verfahren der Rektifikationskolonne zugeführte Flüssigkeit II, bezogen auf die in ihr enthaltene Gesamtmenge an Acrolein und Methacrolein, nicht mehr als 1000 Gew.-%, häufig nicht mehr als 900 Gew.-%, vielfach nicht mehr als 800 Gew.-%, oft nicht mehr als 700 Gew.-%, teilweise nicht mehr als 600 Gew.-%, manchmal nicht mehr als 500 Gew.-% und unter Umständen nicht mehr als 400 Gew.-% an Aceton enthalten.

Dies ist nicht zuletzt darauf zurückzuführen, dass Aceton im Rahmen der relevanten Partialoxidationen von C₃- und/oder C₄-Vorläuferverbindungen von Acrylsäure und/oder Methacrylsäure nicht in großen Mengen als Nebenprodukt gebildet wird. Für eine Verwirklichung des erfindungsgemäßen Verfahrens ohne Zusatz von externem (d.h., nicht aus der zur gewünschten Acrylsäure und/oder Methacrylsäure führenden partiellen heterogen katalysierten Gasphasenoxidation selbst entstammendem) Aceton in die Flüssigkeit II bedarf es daher einer besonders sorgfältigen Wahl der Rahmenbedingungen sowohl für die partielle Gasphasenoxidation als auch für die Überführung der im Produktgasgemisch derselben enthaltenen Acrylsäure und/oder Methacrylsäure in die kondensierte Phase.

Vorzugsweise kann eine relative Anreicherung des Aceton durch eine geschickte Wahl der Multimetalloxidkatalysatoren für die mit Vorteil von Propylen bzw. iso-Buten ausgehend in zweckmäßiger Weise zweistufig durchgeführte Partialoxidation der Acrylsäure- und/oder Methacrylsäure-Vorläuferverbindungen erzielt werden. Wenige orientierende Versuche für eine entsprechende Auswahl sind diesbezüglich für den Fachmann ausreichend. Erhöhte Reaktionstemperaturen fördern in der Regel die Acetonbildung. Desgleichen gilt für erhöhte Gehalte der jeweiligen Vorläuferverbindung im Reaktionsgasausgangsgemisch sowie für eine niedrige spezifische Wärme der angewandten inerten Verdünnungsgase. Ferner ist es günstig, die Belastung des Katalysatorbetts (erfindungsgemäß vorteilhaft ein Katalysatorfestbett) mit der jeweiligen Vorläuferverbindung ≥ 120 Nl/l•h, bzw. ≥ 130 Nl/l•h, bzw. ≥ 140 Nl/l•h, bzw. ≥ 150 Nl/l•h, bzw. ≥ 160 Nl/l•h zu wählen. In der Regel wird vorgenannte Belastung des Katalysatorfestbetts ≤ 300 Nl/l•h, häufig ≤ 250 Nl/l•h und vielfach ≤ 200 Nl/l•h betragen.

Wird zur Herstellung von Acrylsäure von Propylen ausgegangen, wird man in der ersten Reaktionsstufe (Propylen zu Acrolein) vorzugsweise Mo, Bi und Fe enthaltende Multimetalloxidkatalysatoren verwenden. Als solche kommen insbesondere jene der EP-A 015 565, der EP-A 575 897, der DE-A 197 46 210 und der DE-A 198 55 913 in Betracht. Diese Multimetalloxidkatalysatoren eignen sich gleichermaßen für die erste Oxidationsstufe einer gasphasenkatalytisch oxidativen Herstellung von Methacrolein aus iso-Buten. In der zweiten Oxidationsstufe (Acrolein zu Acrylsäure) wird man mit Vorteil Mo und V enthaltende Multimetalloxidkatalysatoren einsetzen. Als solche kommen insbesondere jene der DE-A 100 46 925, DE-A 198 15 281, DE-A 43 35 973, EP-A 714 700, EP-A 668 104, DE-A 197 36 105, DE-A 197 40 493 und der DE-A 195 28 646 in Betracht.

Erfindungsgemäß günstig beträgt der Acroleinumsatz in der zweiten Oxidationsstufe (Acrolein zu Acrylsäure) 99,0 bis 99,9 mol-%, vorzugsweise 99,3 bis 99,9 mol-% und besonders bevorzugt 99,6 bis 99,9 mol-%.

Vorgenannte Höchstwerte sind z.B. dann erreichbar, wenn die zweite Reaktionsstufe wenigstens einen Nachreaktor gemäß der DE-A 10 2004 021 764 bzw. DE-A 10 2004 021 763 umfasst. Alternativ können Verfahrensvarianten gemäß dem in diesen Schriften genannten Stand der Technik angewendet werden.

Der Propylenumsatz in der ersten Oxidationsstufe wird mit Vorteil zu ≥ 94 mol-%, und ≤ 99 mol-% gewählt. Der DE-A 25 26 238, EP-A 092 097, EP-A 058 927, DE-A 41 32 263, DE-A 41 32 684 und DE-A 40 22 212 können Multimetalloxidkatalysatoren (die vorzugsweise Mo und P enthalten) für die zweite Oxidationsstufe _{"}Methacrolein zu Methacrylsäure" entnommen werden.

Zwar können zur Erzeugung von für erfindungsgemäße Verfahren geeigneten Flüssigkeiten II auch die in dieser Schrift bereits erwähnten organischen Absorptionsmittel verwendet werden, um die Acrylsäure und/oder Methacrylsäure aus der Gasphase des Reaktionsgasgemischs der Partialoxidation in die kondensierte Phase überzuführen. Erfindungsgemäß bevorzugt erfolgt diese Überführung in die kondensierte Phase jedoch durch fraktionierende Kondensation des Reaktionsgasgemischs (in sich selbst aufsteigend in trennwirksame Einbauten enthaltenden Trennkolonnen) und/oder durch Absorption in Wasser bzw. wässrige Lösungen (bevorzugt im Gegenstrom).

Dies deshalb, weil Aceton eine besonders hohe Affinität zu Wasser aufweist (sei es das entgegenströmende kondensierte Reaktionswasser und/oder entgegenströmendes zu Absorptionszwecken aufgegebenes externes Wasser bzw. externe wässrige Lösung (sie sollte wenigstens zu 80 Gew.-%, vorzugsweise zu wenigstens 90 Gew.-% und besonders bevorzugt zu wenigstens 95 Gew.-% aus Wasser bestehen)). Geringe Mengen an wässrigem Absorptionsmittel sind erfindungsgemäß bevorzugt, weil sie die Relativanreicherung des Aceton verstärken.

Erfindungsgemäß günstige wässrige Absorptionsmittelmengen können z.B. 5 bis 10 Liter je Nm³ Reaktionsgasgemisch betragen.

Beispielsweise eignen sich als wässrige Absorptionsmittel wässrige Lösungen (die dem Verfahren entnommen sein können), die enthalten:

| | |
|---|---|
| 0,1 bis 5 Gew.-% | Acrylsäure, |
| 0,1 bis 10 Gew.-% | Essigsäure und |
| 80 bis 99,8 Gew.-% | Wasser. |

Im Sinn einer Relativanreicherung des Acetons ist es erfindungsgemäß außerdem vorteilhaft, wenn am Kopf der trennwirksame Einbauten enthaltenden Trennkolonne die dem in ihr aufsteigenden Reaktionsgasgemisch entgegenströmende wässrige Phase eine Temperatur von 60 bis 90°C aufweist. Darunter befindliche Temperaturen sind im Sinne einer Relativanreicherung des Acetons im Absorbat erfindungsgemäß weniger bevorzugt.

Eine weitere Relativanreicherung des Aceton kann dadurch bewirkt werden, dass man das erhaltene wässrige Absorbat einer Desorption und/oder Strippung mit Luft, Stickstoff und/oder einem sonstigen Inertgas unter geeigneten Bedingungen unterwirft. Dabei ist es vorteilhaft, wenn sowohl die Sumpf- als auch die Kopftemperatur in der dazu verwendeten, trennwirksame Einbauten enthaltenden, Trennkolonne nicht zu hoch ist. Erfindungsgemäß günstig betragen vorgenannte Temperaturen von 50 bis 75°C. Weiterhin ist es im erfindungsgemäßen Sinn vorteilhaft, wenn der dabei angewandte Kopfdruck nicht zu niedrig gewählt wird. Bevorzugte Kopfdrucke liegen bei 600 mbar bis zu 1 bar.

Sollte die auf dem beschriebenen Weg erzielte Relativanreicherung des Aceton nicht ausreichend sein (eine weitere Anreicherungsmöglichkeit besteht in einer Vorabrektifikation bei tiefen Temperaturen und nicht zu niedrigen Drucken), kann der erfindungsgemäß zu behandelnden Flüssigkeit zusätzlich Aceton zugesetzt werden. Diese Maßnahme ist insofern vergleichsweise unbedenklich, als eine im weiteren Verlauf der Abtrennung von Acrylsäure und/oder Methacrylsäure angestrebte Abtrennung des Aceton rektifikativ mühelos erreichbar ist.

D.h., die Anzahl der zum Erreichen von Reinacrylsäure und/oder Reinmethacrylsäure (häufig auch als _{"}glacial acrylic acid" bzw. _{"}glacial methacrylic acid") erforderlichen Trennschritte wird beim erfindungsgemäßen Verfahren nicht erhöht (normalerweise enthalten vorgenannte Reinsäuren ≥ 99,8 Gew.-% an Acryl- und/oder Methacrylsäure).

Wesentlich am erfindungsgemäßen Verfahren ist, dass es insbesondere auf Flüssigkeiten II anwendbar ist, die erzeugt wurden, ohne Reinacrylsäure Acrolein und Aceton als Reinsubstanz (normalerweise Reinheit ≥ 99,8 Gew.-%) zuzusetzen.

Weiterhin ist das erfindungsgemäße Verfahren auf Flüssigkeiten II anwendbar, die erzeugt wurden, ohne Reinmethacrylsäure Methacrolein und Aceton als Reinsubstanz zuzusetzen.

Insbesondere ist das erfindungsgemäße Verfahren auch dann auf Flüssigkeiten II anwendbar, wenn das Verhältnis der in ihr enthaltenen Gesamtgewichtsmenge an Acrolein und Methacrolein zur in ihr enthaltenen Gewichtsmenge an Aceton nicht 0,95 bis 1,07 beträgt.

Auch ist das erfindungsgemäße Verfahren auf Flüssigkeiten II anwendbar, in denen das Gewichtsverhältnis von enthaltenem Acrolein zu Acetaldehyd nicht 10:1 und nicht 12:1, oder nicht 10:1 bis zu 12:1 beträgt. Dies gilt insbesondere dann, wenn in der Flüssigkeit II das Gewichtsverhältnis von enthaltenem Acrolein zur enthaltenem Aceton nicht gleichzeitig (oder nur) 5:1 bzw. 3,5:1 oder nicht gleichzeitig (oder nur) 3,5:1 bis 5:1 beträgt.

Das erfindungsgemäße Verfahren ist insbesondere auf Flüssigkeiten II anwendbar, deren Gehalt an Acrylsäure und/oder Methacrylsäure, bezogen auf das Gesamtgewicht der Flüssigkeit II, ≥ 20 Gew.-%, oder ≥ 30 Gew.-%, oder ≥ 40 Gew.-%, oder ≥ 50 Gew.-%, oder ≥ 60 Gew.-%, oder ≥ 70 Gew.-%, oder ≥ 80 Gew.-%, oder ≥ 90 Gew.-%, oder ≥ 95 Gew.-%, oder ≥ 98 Gew.-% beträgt.

In der Regel wird der Gehalt der erfindungsgemäß zu behandelnden Flüssigkeiten II an Acrylsäure und/oder Methacrylsäure ≤ 99 Gew.-% betragen.

D.h., das erfindungsgemäße Verfahren eignet sich z.B. für Flüssigkeiten II, die 20 bis 65 Gew.-%, oder 30 bis 65 Gew.-%, oder 40 bis 65 Gew.-%, oder 50 bis 65 Gew.-%, bezogen auf das Gesamtgewicht der Flüssigkeit 11 an Acrylsäure und/oder Methacrylsäure enthalten.

Es eignet sich aber auch für Flüssigkeiten II, die 70 bis 95 Gew.-%, oder 72 bis 90 Gew.-%, oder 75 bis 85 Gew.-%, bezogen auf das Gesamtgewicht der Flüssigkeit II an Acrylsäure und/oder Methacrylsäure enthalten.

Insbesondere eignet sich das erfindungsgemäße Verfahren für Flüssigkeiten II (die Wasser enthalten), deren Gehalt an Wasser, bezogen auf das Gesamtgewicht der Flüssigkeit II, weniger oder mehr als 30 Gew.-% (oder = 30 Gew,-%) beträgt. D.h., das erfindungsgemäße Verfahren ist auf Flüssigkeiten II anwendbar, deren wie vorstehend beschriebener Wassergehalt 5 bis 28 Gew.-%, oder 10 bis 25 Gew.-%, oder 15 bis 20 Gew.-% beträgt. Es ist aber auch auf Flüssigkeiten II anwendbar, deren wie vorstehend beschriebener Wassergehalt 32 bis 85 Gew.-%, oder 35 bis 80 Gew.-%, oder 40 bis 75 Gew.-%, oder 45 bis 70 Gew.-%, oder 50 bis 65 Gew.-%, oder 55 bis 60 Gew.-% beträgt. Anstelle von Wasser können die vorgenannten Flüssigkeiten II aber auch Mischungen aus 70 bis 75 Gew.-% Diphenylether und 25 bis 30 Gew.-% Diphenyl enthalten. Selbstredend können solche Wasservertreter aber auch Mischungen aus 70 bis 75 Gew.-% Diphenylether und 25 bis 30 Gew.-% Diphenyl, sowie, bezogen auf diese Mischungen, 0,1 bis 25 Gew.-% Dimethylphthalat sein.

Ferner kommen als solche Wasservertreter auch Gemische aus Wasser und einem azeotropen Schleppmittel wie z. B. Heptan, Dimethylcyclohexan, Ethylcyclohexan, Toluol, Ethylbenzol, Chlorbenzol, Xylol oder Gemische aus diesen Schleppmitteln in Betracht.

In der Regel wird die erfindungsgemäß zu behandelnde Flüssigkeit II, bezogen auf die in der Flüssigkeit II enthaltene Menge an Acrylsäure und/oder Methacrylsäure, wenigstens 10 gew.ppm an Acrolein und/oder Methacrolein enthalten. Häufig wird der vorgenannte Gehalt an Acrolein und/oder Methacrolein beim erfindungsgemäßen Verfahren wenigstens 20 gew.ppm, oder wenigstens 30 gew.ppm, oder wenigstens 40 gew.ppm, oder wenigstens 50 gew.ppm, oder wenigstens 60 gew.ppm, oder wenigstens 70 gew.ppm, oder wenigstens 80 gew.ppm, oder wenigstens 90 gew.ppm, oder wenigstens 100 gew.ppm betragen.

Vielfach wird der vorgenannte Gehalt an Acrolein und/oder Methacrolein wenigstens 150 gew.ppm, oder wenigstens 250 gew.ppm, oder wenigstens 300 gew.ppm, oder wenigstens 400 gew.ppm, oder wenigstens 500 gew.ppm betragen. In der Regel wird der vorgenannte Gehalt an Acrolein und/oder Methacrolein in der erfindungsgemäß zu behandelnden Flüssigkeit II jedoch ≤ 20000 gew.ppm, vielfach ≤ 15000 gew.ppm, häufig ≤ 10000 gew.ppm, und oft sogar ≤ 7500 gew.ppm bzw. ≤ 5000 gew.ppm betragen.

D. h., der Gesamtgehalt der erfindungsgemäß zu behandelnden Flüssigkeit 11 an Acrolein und/oder Methacrolein sowie Aceton wird, bezogen auf ihr Gesamtgewicht, vielfach ≥ 10 gew.ppm bis ≤ 4 Gew.-%, oder ≥ 20 gew.ppm bis ≤ 3 Gew.-%, oder ≥ 30 gew.ppm bis ≤ 2,5 Gew.-%, oder ≥ 40 gew.ppm bis ≤ 2 Gew.-%, oder ≥ 50 gew.ppm bis ≤ 1,5 Gew.-%, oder ≥ 75 gew.ppm bis ≤ 1 Gew.-%, oder ≥ 100 gew.ppm bis ≤ 0,5 Gew.-% betragen.

Neben Acrolein und/oder Methacrolein kann die erfindungsgemäß zu behandelnde Flüssigkeit II zusätzlich andere Aldehyde wie z. B. Benzaldehyd und Furfurale enthalten. Bezogen auf die in der Flüssigkeit II enthaltene Menge an Acrylsäure und/oder Methacrylsäure kann der Gehalt der Flüssigkeit II an Benzaldehyd und/oder Furfuralen (Furfural-2 und/oder Furfural-3) jeweils 10 gew.ppm bis 10000 gew.ppm, bzw. 20 gew.ppm bis 5000 gew.ppm, bzw. 50 bis 3000 gew.ppm betragen.

Auch kann die Flüssigkeit II beim erfindungsgemäßen Verfahren zusätzlich noch niedere Aldehyde wie Formaldehyd, Glyoxal, Acetaldehyd, Propionaldehyd, n-Butyraldehyd, iso-Butyraldehyd und Crotonaldehyd enthalten. Deren Mengenanteile können dabei von einer vergleichbaren Größenordnung wie die vorgenannten sein.

Mit Vorteil enthält die beim erfindungsgemäßen Verfahren der Reaktionskolonne zugeführte Flüssigkeit II, bezogen auf die in ihr enthaltene Gesamtmenge an Aldehyden wenigstens 10 Gew.-%, oder wenigstens 15 Gew.-%, mit Vorteil wenigstens 20 Gew.-%, vorteilhafter wenigstens 25 Gew.-%, besser wenigstens 30 Gew.-%, noch besser wenigstens 35 Gew.-%, mit Vorzug wenigstens 40 Gew.-%, besonders bevorzugt wenigstens 45 Gew.-% und ganz besonders bevorzugt wenigstens 50 Gew.-% an Aceton. Üblicherweise wird der vorgenannte Acetongehalt jedoch nicht mehr als 500 Gew.-% betragen. Dabei kann der Aldehydgehalt z. B. dadurch gezielt abgesenkt werden, dass man der Flüssigkeit II Wirkstoffe zusetzt, die die Aldehyde selektiv chemisch binden.

Selbstredend werden alle in dieser Schrift angesprochenen Verfahrensstufen, bei denen Acrylsäure und/oder Methacrylsäure in flüssiger Phase enthalten ist, im Beisein von Polymerisationsinhibitoren durchgeführt. Als solche kommen insbesondere Hydrochinon, Phenothiazin (PTZ), Hydrochinonmonomethylether (MEHQ) sowie N-Oxyl-Radikale (vgl. US-A 2004/0242826) in Betracht.

Unter den letzteren sind vor allem das 4-Hydroxy-2,2,6,6-tetramethylpiperidin-N-oxyl und das 2,2,6,6-Tetramethyl-piperidin-N-oxyl (bzw. deren Gemisch) bevorzugt.

Vorgenannte Polymerisationsinhibitoren können jeweils für sich oder auch in den unterschiedlichsten Gemischen zum Einsatz kommen. Üblicherweise werden sie in einer Gesamtmenge von 100 bis 5000, vorzugsweise 200 bis 1000 gew.ppm, bezogen auf das Gewicht der Acrylsäure und/oder Methacrylsäure enthaltenden Flüssigphase, eingesetzt. Selbstverständlich können vorgenannte Polymerisationsinhibitoren aber auch als Bestandteil von anderen Polymerisationsinhibitorsystemen verwendet werden. In der Regel wird im Rahmen der erfindungsgemäßen Rektifikation auch molekularer Sauerstoff als Polymerisationsinhibitor mitverwendet. In einfacher Weise ist dies dadurch möglich, dass man einen Luftstrom durch die Rektifikationskolonne führt.

Zusätzlich zur Flüssigkeit II kann beim erfindungsgemäßen Verfahren auch die Rücklaufflüssigkeit in der Rektifikationskolonne polymerisationsinhibiert werden.

Die Anreicherung der Acrylsäure und/oder Methacrylsäure kann bei der erfindungsgemäßen rektifikativen Auftrennung von Flüssigkeiten II sowohl am Kopf als auch im Sumpf der Rektifikationskolonne erfolgen. Selbstredend können aber auch im oberen, unteren oder mittleren Teil der Rektifikationskolonne Acrylsäure und/oder Methacrylsäure angereichert enthaltende Fraktionen entnommen werden.

Die in der Rektifikationskolonne beim erfindungsgemäßen Verfahren normalerweise enthaltenen trennwirksamen Einbauten verfolgen beim erfindungsgemäßen Verfahren den Zweck, die Oberfläche für den die Auftrennung in der Trennkolonne bewirkenden Wärme- und Sauerstoffaustausch zu erhöhen. Als solche Einbauten kommen z. B. Packungen, Füllkörper und/oder Stoffaustauschböden in Betracht. Letztere können z. B. Siebböden (Zwangssiebböden oder Dual-Flow (Regensiebböden)-Böden) und/oder Glockenböden sein.

Stoffaustauschböden, auf denen Gleichgewicht herrscht zwischen ablaufender Flüssigkeit und aufsteigendem Dampf werden theoretische Böden bezeichnet. Dieser Begriff lässt sich auf alle anderen für Gegenstromrektifikationen geeigneten trennwirksamen Einbauten übertragen (z.B. Packungen und Füllkörperschüttungen).

In dieser Schrift wird deshalb in zweckmäßiger Weise ganz allgemein von theoretischer Trennstufe gesprochen. Dabei wird als theoretische Trennstufe diejenige Raumeinheit definiert, die eine Anreicherung entsprechend dem thermodynamischen Gleichgewicht bewirkt.

Die Zufuhr der Flüssigkeit II in die Rektifikationskolonne kann, bezogen auf die Gesamtzahl der theoretischen Trennstufen (und von unten nach oben betrachtet), sowohl im unteren Drittel als auch im oberen Drittel der Rektifikationskolonne erfolgen. In entsprechender Weise bezogen kann sie auch unterhalb oder oberhalb der Mitte der Rektifikationskolonne erfolgen. Selbstredend kann die Zufuhr der Flüssigkeit II in die Rektifikationskolonne auch am Kolonnenkopf unterhalb der Kopfproduktentnahme erfolgen.

Erfindungsgemäß zweckmäßig befinden sich oberhalb der Zufuhrstelle der Flüssigkeit II in die Rektifikationskolonne beim erfindungsgemäßen Verfahren wenigstens zwei, bzw. wenigstens vier, bzw. wenigstens sechs, bzw. wenigstens acht, bzw. wenigstens zehn, bzw. wenigstens zwölf theoretische Trennstufen.

Prinzipiell kann die Zufuhr der Flüssigkeit II in die Rektifikationskolonne beim erfindungsgemäßen Verfahren auch unmittelbar in den Sumpf der Rektifikationskolonne erfolgen. Das Rücklaufverhältnis kann beim erfindungsgemäßen Verfahren in weiten Bereichen variieren. Es kann z.B. 2:1 (zweifacher Rücklauf relativ zur Entnahmemenge) oder mehr oder weniger betragen.

Vorzugsweise wird das erfindungsgemäße Verfahren bei reduziertem Druck durchgeführt. Erfindungsgemäß besonders geeignete Kopfdrucke betragen ≤ 500 mbar, besonders bevorzugt 10 bis 500 mbar, häufig 10 bis 200 mbar und vorzugsweise 10 bis 190 bzw. bis 150 mbar. Der Druckverlust über die Rektifikationskolonne beträgt beim erfindungsgemäßen Verfahren vorteilhaft 300 bis 100, oder 250 bis 150 mbar.

Die Temperatur im Sumpf der Rektifikationskolonne beträgt beim erfindungsgemäßen Verfahren zweckmäßig 50 bis 230°C, vorzugsweise 70 bis 210°C, bzw. 90 bis 190°C oder 110 bis 170°C.

Unter einer Flüssigkeit II an der Zufuhrstelle Z in die Rektifikationskolonne soll beim erfindungsgemäßen Verfahren ein Stoffstrom verstanden werden, der zu mehr als 80%, vorzugsweise zu mehr als 85%, oder zu mehr als 90%, oder zu mehr als 95%, oder zu mehr als 99% seines Gesamtvolumens als kondensierte Phase vorliegt.

Im übrigen kann das erfindungsgemäße Verfahren z.B. wie die in den Schriften DE-A 103 47 664 und DE-A 103 00 816 beschriebenen rektifikativen Verfahren durchgeführt werden. Im erfindungsgemäßen Verfahren abgetrennte Acrylsäure und/oder Methacrylsäure kann in sich an das erfindungsgemäße Verfahren anschließenden Verfahren zur Herstellung von Polymerisaten in solche Polymerisate radikalisch einpolymerisiert werden.

### Beispiele

A) Es wurden 4 nicht unterscheidbare Proben von jeweils 0,5 ml frisch zubereitet (Destillation, dann einfrieren), deren Gehalt an Acrylsäure > 99,8 Gew.-% und deren Aldehyd/Keton-Gehalt < 10 gew.ppm betrug. Jede der Proben wurde durch Zusatz von 25 gew.ppm Phenothiazin polymerisationsinhibiert. Dann wurden durch Zusatz von Aceton und/oder Acrolein die nachfolgenden Gehalte der einzelnen Proben eingestellt:
Probe 1: kein Zusatz;
Probe 2: 1000 gew.ppm Acrolein;
Probe 3: 10000 gew.ppm Acrolein;
Probe 4: 1000 gew.ppm Acrolein und 1000 gew.ppm Aceton.

Unter Luftatmosphäre wurde jede der Proben in eine 1,8 ml Glasampulle überführt. Unmittelbar nach Fertigstellung wurden die Ampullen bei 120°C im Umlufttrockenschrank drehend gelagert, um vollständige Durchmischung zu gewährleisten.
Dann wurde die Zeit T bis zur vollständigen Polymerisation der jeweiligen Probe visuell erfasst.
Die Versuchsreihe wurde dreimal wiederholt und dabei folgende Wert T [in Minuten] erhalten:

| | Versuch 1 | Versuch 2 | Versuch 3 | Mittelwert |
|---|---|---|---|---|
| Probe 1 | 479 | 483 | 480 | 481 |
| Probe 2 | 299 | 283 | 294 | 292 |
| Probe 3 | 111 | 111 | 105 | 109 |
| Probe 4 | 323 | 321 | 327 | 324 |

B) In entsprechender Weise wie in A) wurden 4 nicht unterscheidbare Proben von jeweils 0,5 ml frisch zubereitet, deren Gehalt an Methacrylsäure > 99,8 Gew.-% und deren Aldehyd/Keton-Gehalt > 10 gew.ppm betrug. Jede der 4 Proben wurde durch Zusatz von 10 gew.ppm Phenothiazin polymerisationsinhibiert. Durch Zusatz von Aceton und/oder Methacrolein wurden die nachfolgenden Gehalte der einzelnen Proben eingestellt:
Probe 1: kein Zusatz;
Probe 2: 10000 gew.ppm Methacrolein;
Probe 3: 400 gew.ppm Aceton;
Probe 4: 1000 gew.ppm Methacrolein + 1000 gew.ppm Aceton.

Dann wurde wie unter A) beschrieben die Zeit T (in Minuten) bis zur vollständigen Polymerisation der jeweiligen Probe bestimmt. Die Versuchsreihe wurde gleichfalls dreimal wiederholt und dabei folgende Werte T [in Minuten] erhalten:

| | Versuch 1 | Versuch 2 | Versuch 3 | Mittelwert |
|---|---|---|---|---|
| Probe 1 | 239 | 263 | 269 | 257 |
| Probe 2 | 190 | 200 | 200 | 197 |
| Probe 3 | 288 | 301 | 319 | 303 |
| Probe 4 | 288 | 288 | 310 | 295 |

C) Es wurden folgende Rektifikationsversuche durchgeführt:
I. Kopfdosierung
Die verwendete Rektifikationsvorrichtung umfasste:
- einen Dreihalskolben mit einem Fassungsvermögen von 250 ml;
- eine Vigreux-Kolonne der Länge 32 cm, die auf einer Länge von 21 cm gegen die Umgebung mittels Vakuum thermisch isoliert war;
- ein Ölbad zur Temperierung des Dreihalskolbens;
- ein Nadelventil samt Zulaufkapillare zum Zweck der Kopfdosierung;
- eine in die Flüssigphase im Dreihalskolben ragende Kapillare zum Zweck der Zudosierung von Luft;
- ein Thermoelement zur Ermittlung der Kopftemperatur und ein Thermometer zur Ermittlung der Sumpftemperatur;

Wie in A) wurden 100 g einer Acrylsäure mit einem Acrylsäuregehalt > 99,8 Gew.-% und einem Aldehyd/Keton-Gehalt < 10 gew.ppm frisch zubereitet (Destillation; dann einfrieren) und anschließend mit 100 gew.ppm Phenothiazin (PTZ) versetzt und in den Dreihalskolben vorgelegt.
Weitere 60 g derselben mit 100 gew.ppm Phenothiazin versetzten Acrylsäure wurden mit unterschiedlichen Mengen an Acrolein, Aceton und/oder Acetaldehyd dotiert.
Bei einer Badtemperatur von 100°C wurde im Dreihalskolben eine Temperatur von 87°C erreicht. Beim Anlegen eines Unterdruckes von 133 mbar destillierte die vorgelegte Acrylsäure bei einer Kopftemperatur von 83°C (Temperatur der Gasphase). Es wurde ein Rücklaufverhältnis von 2:1 eingestellt (zweifacher Rücklauf relativ zur Entnahmemenge). Die Menge der entnommenen Acrylsäure wurde durch Kopfdosierung mit der dotierten Acrylsäure ausgeglichen.
Die Rektifikation wurde jeweils während 60 min kontinuierlich betrieben. Anschließend wurde die jeweils gebildete Polymerisatmenge ausgewogen. Polymerisatbildung wurde im Kolonnensumpf und am Kolonnenkopf festgestellt.
In Abhängigkeit von der verwendeten Dotierung wurden die in der nachfolgenden Tabelle 1 aufgelisteten Ergebnisse erhalten :

**Tabelle 1**

| Dotierung [gew.ppm] | | | Polymerisatmenge [mg] | |
|---|---|---|---|---|
| Acrolein | Aceton | Acetaldehyd | am Kopf | im Sumpf |
| 0 | 0 | 0 | 100 | <20 |
| 500 | 0 | 0 | 290 | <30 |
| 1000 | 0 | 0 | 400 | <20 |
| 0 | 1000 | 0 | 80 | <20 |
| 1000 | 1000 | 0 | 300 | <20 |
| 1000 | 2000 | 0 | 80 | <30 |
| 500 | 0 | 500 | 270 | 150 |
| 500 | 1000 | 500 | 110 | 55 |

II. Kolonnenmittedosierung
Der Versuch gemäß I. wurde wiederholt, die Zulaufkapillare wurde jedoch bis zur Kolonnenmitte geführt.
In Abhängigkeit von der verwendeten Dotierung wurden die in der nachfolgenden Tabelle 2 aufgelisteten Ergebnisse erhalten :

**Tabelle 2**

| Dotierung [gew.ppm] | | Polymerisatmenge [mg] | |
|---|---|---|---|
| Acrolein | Aceton | am Kopf | im Sumpf |
| 0 | 0 | 150 | <30 |
| 1000 | 0 | 710 | <25 |
| 0 | 1000 | 110 | <25 |
| 1000 | 1000 | 360 | <25 |
| 1000 | 2000 | 310 | <25 |

III. Dotierte Sumpfvorlage
Der Versuch gemäß I. wurde wiederholt. Im Unterschied zu I. wurde jedoch die Vorlage mitdotiert.
In Abhängigkeit von der angewandten Vorlage- und/oder Kopfzulaufdotierung wurden die in der nachfolgenden Tabelle 3 aufgelisteten Ergebnisse erhalten :

**Tabelle 3**

| Dotierung [gew.ppm] | | | | Polymerisatmenge [mg] | |
|---|---|---|---|---|---|
| Acrolein (Kopf) | Aceton (Kopf) | Acrolein (Vorlage) | Aceton (Vorlage) | am Kopf | im Sumpf |
| 0 | 0 | 1000 | 0 | 240 | <20 |
| 1000 | 0 | 1000 | 0 | 190 | 420 |
| 0 | 0 | 1000 | 1000 | 110 | <25 |
| 0 | 0 | 0 | 1000 | 105 | <25 |
| 1000 | 1000 | 1000 | 1000 | 150 | 100 |

## Patentansprüche

1. Verfahren der rektifikativen Auftrennung einer Flüssigkeit II, deren Gehalt an Acrylsäure und/oder Methacrylsäure, bezogen auf das Gesamtgewicht der Flüssigkeit II, wenigstens 10 Gew.-% beträgt, und die neben Methacrylsäure und/oder Acrylsäure sowohl Acrolein und/oder Methacrolein als auch Aceton in einer Gesamtmenge von nicht mehr als 5 Gew.-%, bezogen auf die in der Flüssigkeit II enthaltene Menge an Acrylsäure und/oder Methacrylsäure mit der Maßgabe enthält, dass das Gewichtsverhältnis von in der Flüssigkeit 11 enthaltenem Acrolein zu in der Flüssigkeit 11 enthaltenem Aceton von 3,5 verschieden ist, und die erzeugt wurde, ohne einer anderen Acrylsäure und/oder Methacrylsäure enthaltenden Flüssigkeit I Acrolein oder Methacrolein als Reinsubstanz zuzusetzen, **dadurch gekennzeichnet, dass** man die Flüssigkeit II mit der Maßgabe in eine Rektifikationskolonne führt, dass sie, bezogen auf die in der Flüssigkeit II enthaltene Gesamtmenge an Acrolein und Methacrolein, wenigstens 10 Gew.-% Aceton enthält.

2. Verfahren nach Anspruch 1, **dadurch gekennzeichnet, dass** die in die Rektifikationskolonne geführte Flüssigkeit 11, bezogen auf die Gesamtmenge an in der Flüssigkeit II enthaltenem Acrolein und Methacrolein, wenigstens 20 Gew.-% Aceton enthält.

3. Verfahren nach Anspruch 1, **dadurch gekennzeichnet, dass** die in die Rektifikationskolonne geführte Flüssigkeit II, bezogen auf die Gesamtmenge an in der Flüssigkeit 11 enthaltenem Acrolein und Methacrolein, wenigstens 30 Gew.-% Aceton enthält.

4. Verfahren nach einem der Ansprüche 1 bis 3, **dadurch gekennzeichnet, dass** der Gehalt der Flüssigkeit 11 an Acrylsäure und/oder Methacrylsäure 20 bis 99 Gew.-% beträgt.

5. Verfahren nach einem der Ansprüche 1 bis 3, **dadurch gekennzeichnet, dass** der Gehalt der Flüssigkeit II an Acrylsäure und/oder Methacrylsäure 20 bis 65 Gew.-% oder 70 bis 95 Gew.-% beträgt.

6. Verfahren nach einem der Ansprüche 1 bis 5, **dadurch gekennzeichnet, dass** die Flüssigkeit II Wasser enthält.

7. Verfahren nach einem der Ansprüche 1 bis 6, **dadurch gekennzeichnet, dass** die Flüssigkeit II mehr als 30 Gew.-% an Wasser enthält.

8. Verfahren nach einem der Ansprüche 1 bis 6, **dadurch gekennzeichnet, dass** die Flüssigkeit II 5 bis ≤ 30 Gew.-% an Wasser enthält.

9. Verfahren nach einem der Ansprüche 1 bis 8, **dadurch gekennzeichnet, dass** die Flüssigkeit II als Polymerisationsinhibitor wenigstens ein N-Oxyl-Radikal zugesetzt enthält.

10. Verfahren nach einem der Ansprüche 1 bis 9, **dadurch gekennzeichnet, dass** sich an das Verfahren der rektifikativen Auftrennung ein Verfahren zur Herstellung von Polymerisaten anschließt, bei dem rektifikativ abgetrennte Acrylsäure und/oder Methacrylsäure in solche Polymerisate radikalisch einpolymerisiert wird.

## Claims

1. A process for rectificatively separating a liquid II whose content of acrylic acid and/or methacrylic acid, based on the total weight of the liquid II, is at least 10% by weight, and which, in addition to methacrylic acid and/or acrylic acid, comprises both acrolein and/or methacrolein and acetone in a total amount of not more than 5% by weight, based on the amount of acrylic acid and/or methacrylic acid present in the liquid II, with the proviso that the weight ratio of acrolein present in the liquid II to acetone present in the liquid II is different from 3.5, and which has been generated without adding acrolein or methacrolein as a pure substance to another liquid I comprising acrylic acid and/or methacrylic acid, which comprises conducting the liquid II into a rectification column with the proviso that it comprises, based on the total amount of acrolein and methacrolein present in the liquid II, at least 10% by weight of acetone.

2. The process according to claim 1, wherein the liquid II conducted into the rectification column, based on the total amount of acrolein and methacrolein present in the liquid II, comprises at least 20% by weight of acetone.

3. The process according to claim 1, wherein the liquid II conducted into the rectification column, based on the total amount of acrolein and methacrolein present in the liquid II, comprises at least 30% by weight of acetone.

4. The process according to any of claims 1 to 3, wherein the content in the liquid II of acrylic acid and/or methacrylic acid is from 20 to 99% by weight.

5. The process according to any of claims 1 to 3, wherein the content in the liquid II of acrylic acid and/or methacrylic acid is from 20 to 65% by weight or from 70 to 95% by weight.

6. The process according to any of claims 1 to 5, wherein the liquid II comprises water.

7. The process according to any of claims 1 to 6, wherein the liquid II comprises more than 30% by weight of water.

8. The process according to any of claims 1 to 6, wherein the liquid II comprises from 5 to ≤ 30% by weight of water.

9. The process according to any of claims 1 to 8, wherein the liquid II comprises, as a polymerization inhibitor, at least one added N-oxyl radical.

10. The process according to any of claims 1 to 9, wherein the process for rectificative separation is followed by a process for preparing polymers in which rectificatively removed acrylic acid and/or methacrylic acid is free-radically polymerized to such polymers.

## Revendications

1. Procédé de séparation rectificative d'un liquide II, dont la teneur en acide acrylique et/ou en acide méthacrylique, par rapport au poids total du liquide II, est d'au moins 10 % en poids, et qui contient, outre l'acide méthacrylique et/ou l'acide acrylique, également de l'acroléine et/ou de la méthacroléine, ainsi que de l'acétone, en une quantité totale inférieure ou égale à 5 % en poids, par rapport à la quantité d'acide acrylique et/ou d'acide méthacrylique contenue dans le liquide II, à condition que le rapport de poids entre l'acroléine contenue dans le liquide II et l'acétone contenue dans le liquide II soit différent de 3,5, et qui est généré sans ajouter d'acroléine ou de méthacroléine sous la forme de substance pure à un autre liquide I contenant de l'acide acrylique et/ou de l'acide méthacrylique, **caractérisé en ce que** le liquide II est passé dans une colonne de rectification, à condition qu'il contienne au moins 10 % d'acétone, par rapport à la quantité totale d'acroléine et de méthacroléine contenue dans le liquide II.

2. Procédé selon la revendication 1, **caractérisé en ce que** liquide II passé dans la colonne de rectification contient au moins 20 % en poids d'acétone, par rapport à la quantité totale d'acroléine et de méthacroléine contenue dans le liquide II.

3. Procédé selon la revendication 1, **caractérisé en ce que** liquide II passé dans la colonne de rectification contient au moins 30 % en poids d'acétone, par rapport à la quantité totale d'acroléine et de méthacroléine contenue dans le liquide II

4. Procédé selon l'une quelconque des revendications 1 à 3, **caractérisé en ce que** la teneur du liquide II en acide acrylique et/ou en acide méthacrylique est de 20 à 99 % en poids.

5. Procédé selon l'une quelconque des revendications 1 à 3, **caractérisé en ce que** la teneur du liquide II en acide acrylique et/ou en acide méthacrylique est de 20 à 65 % en poids ou de 70 à 95 % en poids.

6. Procédé selon l'une quelconque des revendications 1 à 5, **caractérisé en ce que** le liquide II contient de l'eau.

7. Procédé selon l'une quelconque des revendications 1 à 6, **caractérisé en ce que** le liquide II contient plus de 30 % en poids d'eau.

8. Procédé selon l'une quelconque des revendications 1 à 6, **caractérisé en ce que** le liquide II contient de 5 à ≤ 30 % en poids d'eau.

9. Procédé selon l'une quelconque des revendications 1 à 8, **caractérisé en ce qu'**au moins un radical N-oxyle est ajouté au liquide II en tant qu'inhibiteur de polymérisation.

10. Procédé selon l'une quelconque des revendications 1 à 9, **caractérisé en ce qu'**un procédé de fabrication de polymères suit le procédé de séparation rectificative, selon lequel l'acide acrylique et/ou l'acide méthacrylique séparé par rectification est incorporé par polymérisation radicalaire dans de tels polymères.
